# EUROPÄISCHE PATENTSCHRIFT

(11) **EP 1 642 965 B1**
(45) Veröffentlichungstag und Bekanntmachung des Hinweises auf die Patenterteilung: **01.08.2007**
(21) Anmeldenummer: 04023435.3
(22) Anmeldetag: 01.10.2004
(51) Int. Cl.: C12N 5/06, C12N 5/00

(54) **Verwendung von differenzierten Fibroblasten oder einer primären Fibroblastkultur zur Transdifferenzierung in Fett-, Knochen- und Knorpelzellen**
Use of differentiated fibroblasts or of a primary fibtroblast culture for the transdifferentiation of adipocytes, osteoblasts and chondrocytes
Utilisation de fibroblastes différenciés ou d'une culture primaire de fibtroblastes pour la transdifférentiation en adipocytes, ostéoblastes et chondrocytes

(43) Veröffentlichungstag der Anmeldung: 05.04.2006
(73) Patentinhaber: Curacyte Discovery GmbH, 04103 Leipzig (DE)
(72) Erfinder: Krieg, Thomas, 53919 Weilerswist-Metternich (DE); Shephard, Pierre, 78479 Reichenau (DE); Eckes, Beate, 50345 Hürth (DE); Herrmann, Konrad, 04229 Leipzig (DE); Etges, Robert, 04229 Leipzig (DE)
(74) Vertreter: Schüssler, Andrea

(56) Entgegenhaltungen:
- WO-A-00/24866
- WO-A-00/37607
- US-A1- 2002 119 126
- JOANNIDES A ET AL: "Efficient generation of neural precursors from adult human skin: astrocytes promote neurogenesis from skin-derived stem cells" LANCET THE, LANCET LIMITED. LONDON, GB, Bd. 364, Nr. 9429, 10. Juli 2004 (2004-07-10), Seiten 172-178, XP004520464 ISSN: 0140-6736
- DYCE P W ET AL: "Stem cells with multilineage potential derived from porcine skin" BIOCHEMICAL AND BIOPHYSICAL RESEARCH COMMUNICATIONS, ACADEMIC PRESS INC. ORLANDO, FL, US, Bd. 316, Nr. 3, 9. April 2004 (2004-04-09), Seiten 651-658, XP004495936 ISSN: 0006-291X

## Beschreibung

Die Erfindung betrifft ein Verfahren, welches es erstmals erlaubt, aus ausdifferenzierten tierischen, bevorzugt humanen, Fibroblasten bzw. primären Fibroblastenkulturen andere Zellen mesenchymalen Ursprungs, wie Fettzellen (Adipozyten), Knochenzellen (Osteoblasten) und Knorpelzellen (Chondrozyten) zu differenzieren um diese Zellen für therapeutische Belange in der regenerativen Medizin (Zell- und Gewebeersatz) einzusetzen.

Vor über 150 Jahren wurde die Existenz mesenchymaler Stammzellen im Blut und Knochenmark postuliert, nachdem man experimentell nachweisen konnte, dass fibroblastenähnliche Zellen aus dem Blut in Wunden einwandern und an Komponenten des Bindegewebes adhärieren. Das Wissen über die Natur dieser adhärenten Zellen ist aber auch heute noch äußerst lückenhaft. In der Folgezeit konnte sowohl auf mRNA- als auch auf Proteinebene gezeigt werden, dass diese Zellen Proteine der ECM synthetisieren, so z.B. Kollagen Typ I, Kollagen Typ IV, Laminin, Fibronektin und zahlreiche Proteoglykane. Einige dieser Zellen synthetisieren das Faktor VIII-assoziierte Antigen, eine Tatsache, die zu der Vermutung Anlass gab, dass es sich bei diesen Zellen auch um Vorläufer von Endothelzellen handeln könnte. Diese Zellen synthetisieren und sezernieren Zytokine, wie z.B. IL-1, IL-6, IL-7, IL-8, IL-11, den Kolonie-stimulierenden Faktor-1 (CSF-1), GM-CSF und den Stammzell-Faktor (c-kit Ligand).

Seit Beginn der 90iger Jahre gelang es durch Vereinzelung dieser adhärenten mesenchymalen Stammzellen/Vorläuferzellen und spezieller Klonierungstechniken homogenere Zellpopulationen zu gewinnen. Als ein entscheidender Nachteil dieser Arbeiten ist die Tatsache anzusehen, dass alle Arbeitsgruppen nach einem individuellen Protokoll gearbeitet haben, dadurch von ganz unterschiedlichen Ausgangszellpopulationen ausgegangen sind und somit die resultierenden Ergebnisse nur äußerst schlecht vergleichbar waren.

Weiterhin ist heute noch sehr wenig über die molekularen Mechanismen, die zur Differenzierung in die unterschiedlichen mesenchymalen Zelltypen führen, bekannt. Es ist unklar, ob die fraglich einheitliche Population der mesenchymalen Stammzellen zunächst einen gemeinsamen Weg der Differenzierung durchlaufen (Vorläuferzellen), um sich dann auf Grund weiterer Differenzierungsschritte in einen speziellen Phänotyp (Chondrozyt, Fibroblast, Osteoblast, Adipozyt) zu differenzieren, oder ob heute noch nicht bekannte präformierte Vorläuferzellen für diese einzelnen Phänotypen existieren.

Bereits in den 70er Jahren wurde die Umwandlung von Fibroblasten in Fettzellen postuliert (Green and Kehinde, Cell (1976) 7: 105-113; Broad and Ham, Eur. J. Biochem. (1983) 135:33-39). Es wurde berichtet, dass allerdings nur sehr wenig Fibroblasten in der Lage zu sein schienen, sich in Fettzellen zu differenzieren und insgesamt erscheint fraglich, ob tatsächlich von Fibroblasten ausgegangen wurde und nicht von Präadipocyten, einer Vorläuferform der Adipozyten, die morphologisch den Fibroblasten sehr ähnlich sind, was mit dem heutigen Wissen als wahrscheinlich anzusehen ist.

Weiterhin konnte durch neuere experimentelle Befunde gezeigt werden, dass sich in vivo aus hämatopoetischen Stammzellen des Knochenmarks eine Vielzahl von nichthämatopoetischen Zelllinien differenzieren lassen. So konnte unter anderem in vivo gezeigt werden, dass sich Skelettmuskelzellen (Ferrari G et al. Muscle regeneration by bone marrow-derived myogenic progenitors. Science (1998) 279: 1528-1530), Herzmuskelzellen (Jackson KA et al. Regeneration of ischemic cardiac muscle and vascular endothelium by adult stem cells. J Clin Invest (2001) 107: 1395-1402), neuronale Zellen (Mezey E et al. Turning blood into brain: cells bearing neuronal antigens generated in vivo from bone marrow. Science (2000) 290: 1779-1782) und Hepatozyten (Lagasse E et al. Purified hematopoietic stem cells can differentiate into hepatocytes in vivo. Nat Med (2000) 6: 1229-1234; Krause DS et al. Multi-organ, multi-lineage engraftment by a single bone marrow-derived stem cell. Cell (2001) 105: 369-377; Alison MR et al. Hepatocytes from non-hepatic adult stem cells. Nature (2000) 406: 257) aus hämatopoetischen Knochenmarks-Stammzellen entwickeln können.

In neuerer Zeit konnte von verschiedenen Arbeitsgruppen in vivo gezeigt werden, dass nach Transplantation hämatopoetischer Stammzellen diese mit ausdifferenzierten gewebsständigen Zellen fusionierten (Wang X et al. Cell fusion is he principal source of bone-marrow-derived hepatocytes. Nature (2003) 422: 897-901; Alvarez-Dolado et al. Fusion of bone-marrow-derived cells with Purkinje neurons, cardiomyocytes and hepatocytes. Nature (2003) 425: 968-973; Nygren JM et al. Bone marrow-derived hematopoietic cells generate cardiomyocytes at a low frequency through cell fusion, but not transdifferentiation. Nat Med (2004) 10: 494-501).

Neueste in-vivo Untersuchungen belegen eindeutig, dass sowohl die Transplantation autologer Stammzellen des Knochenmarks, als auch Skelettmuskelzellen die Herzfunktion nach einem Infarktgeschehen signifikant verbessert. Die molekularen Mechanismen der Differenzierung der Stammzellen zu Herzmuskelzellen und die "Umwandlung" der Skelettmuskelzellen in Herzmuskelzellen sind jedoch bis heute noch weitestgehend unklar.

Harris et al. konnten zeigen, dass Knochenmarksstammzellen in der Lage sind, in vivo zu unterschiedlichen Epithelzellen der Lunge, Leber und Haut zu differenzieren (Harris RG et al. Lack of fusion requirement for development of bone marrow-derived epithelia. Science (2004) 305: 90-93).

Man geht heute übereinstimmend davon aus, dass sich im Knochenmark Stammzellen/Vorläuferzellen befinden, die sich nicht nur in alle hämatopoetischen Zellen differenzieren lassen, sondern auch solche Zellen vorkommen, die sich in Epithelzellen (Leber, Niere, Lunge, Haut, Gastrointestinal-Trakt) Muskelzellen (Skelettmuskel, Herz), in mesenchymale Zellen (Fibroblasten, Fettzellen, Knochenzellen, Knorpelzellen), in Endothelzellen und in neuronale Zellen differenzieren lassen (Herzog et al. Plasticity of marrow-derived stem cells. Blood (2003) 102: 3483-3493).

In den letzten Jahren sind verstärkte Anstrengungen unternommen worden, die Pluripotenz dieser mesenchymalen Stammzellen für therapeutische Zwecke zu nutzen. So wurden diese Zellen nach der klassischen Adhärenztechnik aus autologem Knochenmark gewonnen, die Zellen über mehrere Verdopplungszeiten passagiert und diese Zellen dann in Gelenke des gleichen Patienten mit degenerativer Arthritis injiziert. Ein weiterer therapeutischer Ansatz bestand darin, die autologen mesenchymalen Stammzellen des Knochenmarks zunächst in vitro zu propagieren und sie danach mittels spezifischer Differenzierungssignale in Chondrozyten zu differenzieren und diese Zellen dann dem Patienten als Gewebeersatz zu transplantieren. Entsprechende Heilungsversuche wurden auch erfolgreich bei Patienten mit Herzinfarktgeschehen und schlecht heilenden Frakturen unternommen.

Für viele Bereiche der Forschung und der Medizin ist es wünschenswert, die Differenzierung von Zellen gezielt beeinflussen zu können. Dies gilt insbesondere für die Differenzierung pluripotenter Stammzellen. Therapeutisch wünschenswert ist es weiterhin, differenzierte Zellen in ein weniger differenziertes Stadium überführen zu können, z.B. um
(a) aus bereits differenzierten Zellen wieder Zellen mit größerer Plastizität erhalten zu können, was den Vorteil hat, dass zum Erhalt pluripotenter Zellen nicht auf embryonale Stammzellen zurückgegriffen werden muss, was - bei menschlichen embryonalen Stammzellen - mit einer Reihe von rechtlichen und ethischen Problemen verbunden ist, oder
(b) andere phänotypische Zellen für die regenerative Medizin bereitstellen zu können.

Leider war es bis heute nicht möglich, aus bereits entdifferenzierten primären Zellen in gewünschtem Maß andere phänotypische Zellen zu gewinnen und es wurde auch bisher nicht davon ausgegangen, dass dies grundsätzlich möglich ist.

Das am längsten bekannte Differenzierungs- oder Funktionsstadium von Fibroblasten ist der Myofibroblast, der sich hauptsächlich durch die Expression von Vimentin und α-smooth-muscle actin und Myofilamenten auszeichnet (Gabbiani G, Chaponnier C, Huttner I. Cytoplasmic filaments and gap junctions in epithelial cells and myofibroblasts during wound healing. J Cell Biol (1978) 76: 561-568). Dieser Differenzierungsschritt, hauptsächlich bei der Wundheilung vorkommend, wird durch die orchestrierte Wirkung löslicher Zytokine, hauptsächlich TGFβ und PDGF, Fibronektin und mechanischen Stress ausgelöst. Bis heute ist noch nicht allgemein entschieden, ob es sich bei Myofibroblasten um eine echte Differenzierung, oder um einen passageren Funktionszustand handelt.

Mehrfach ist in der Vergangenheit die Differenzierung von Fibroblasten in Histiozyten/Makrophagen beschrieben worden (Kaye Gl et al. The colonic pericryptal fibroblast sheath: replication, migration and cytodifferentiation of a mesenchymal cell system in adult tissue. Gastroenterology (1968) 54: 852-865; Kopelovich L. Conversion of human fibroblasts to tissue macrophages by the Snyder-Theilen feline sarcoma virus (ST:FeSV) is associated with the de-novo expression of IL-1 alpha, IL-1 beta, IFN-alpha, TNF-alpha, GM-CSF, and CD4. Eur Cytokine Netw (1991) 2: 99-106). In weiteren Arbeiten wurde auch eine Differenzierung von Monozyten/Makrophagen in Fibroblasten beobachtet (Labat ML et al. Monocytic origin of fibrosis. In vitro transformation of HLA-DR monocytes into neo-fibroblasts: inhibitory effect of all-trans retinoic acid on this process. Biomed Pharmacother (1994) 48: 103-111).

Interessanterweise konnte auch gezeigt werden, dass Makrophagen nach einer Schistosoma mansoni Infektion in Fibroblasten transdifferenzieren können (Bertrand S et al. Transdifferentiation of macrophages into fibroblasts as a result of Schistosoma mansoni infection. Int J Dev Biol (1992) 36: 179-184).

Es existieren weiterhin experimentelle Hinweise, dass Fibroblasten, die mit einem Zellextrakt von Lymphozyten bzw. Nervenzellen inkubiert wurden, den Phänotyp von Lymphozyten bzw. Nervenzellen annahmen (Hakelien AM et al. Reprogramming fibroblasts to express T-cell functions using cell extracts. Nature Biotechnol (2002) 20: 460-466).

Toma et al. konnten kürzlich zeigen, dass aus der Dermis von Nagetieren Precursor-Zellen isolierbar sind, die sich in neuronale und Glia-Zellen, in glatte Muskelzellen und in Adipocyten differenzieren lassen (Toma JG et al. Isolation of multipotent adult stem cells from the dermis of mammalian skin. Nature Cell Biology (2001) 3: 778-784).

Chen et al. beschrieben kürzlich, dass es ihnen mit Hilfe von Reversine, einer heterozyklischen Verbindung, möglich war, aus einer myogenen Mauszelllinie (C2C12) multipotente Progenitor-Zellen zu dedifferenzieren und diese in Adipocyten und Osteoblasten zu differenzieren (Chen S et al. Dedifferentiation of lineagecommitted cells by a small molecule. J Am Chem Soc (2004) 126: 410-411). Es sei hier hervorgehoben, dass Chen et al. eine immortalisierte Zelllinie benutzt haben, die nicht mit den biologischen und biochemischen Charakteristika einer primären Zelle vergleichbar ist.

Für die therapeutischen Bedürfnisse des Bereiches Tissue Engineering ergibt sich die dringende Notwendigkeit autologe multipotente Stammzellen/Vorläuferzellen zu generieren und entweder in vitro oder auch in vivo in die gewünschte Zellart zu differenzieren und dem Patienten zu transplantieren. In allererster Linie kommen hierbei Stammzellen des Knochenmarks in Frage. Bei diesem Vorgehen ist eine operative Knochenmarkpunktion unumgänglich, die bei den meist älteren Patienten erfahrungsgemäß auf eine geringe Compliance stößt.

Vielfältige Ansätze sind unternommen worden, um andere Quellen als das Knochenmark für die Gewinnung von Stammzellen zu nutzen. Bei der Differenzierung von Keratinozyten gelang dies schon seit Jahren, indem als Ursprungsort für diese Zellen die Haarwurzeln dienen. Für die Differenzierung von Knochen- und Knorpelzellen hat sich bis zum heutigen Tage noch keine unkompliziert zu erreichende Quelle an Stammzellen/Vorläuferzellen gefunden.

Somit liegt der vorliegenden Erfindung das technische Problem zugrunde, Ausgangszellen und Verfahren zur Gewinnung von gewünschten differenzierten Zellen, unter Vermeidung der Verwendung von Stammzellen, zur Verfügung zu stellen.

Die Lösung dieses technischen Problems erfolgte durch die Bereitstellung der in den Patentarisprüchen gekennzeichneten Ausführungsformen.

Es wurde überraschenderweise gefunden, dass das vorstehende technische Problem durch die Verwendung von aus Hautbioptaden gewonnenen Fibroblasten als Ausgangszellen und unter bestimmten Kultivierungsbedingungen gelöst werden kann.

Bei den zu der vorliegenden Erfindung führenden Experimenten wurde davon ausgegangen, dass schon ausdifferenzierte tierische Zellen, wie z.B. Fibroblasten, unter bestimmten regulativen Bedingungen eine De- oder Transdifferenzierung durchmachen können und sich dann in einen anderen Phänotyp, wie z.B. Fettzellen, Knorpel- oder Knochenzellen differenzieren lassen. In wiederholten Experimenten konnte klar gezeigt werden, dass ein Teil der über Biopsie gewonnenen und kultivierten Fibroblasten, ähnlich wie adulte mesenchymale Stammzellen, in die o.g. Phänotypen (Adipocyten, Chondrocyten, Osteoblasten) zu differenzieren sind.

Somit konnte erstmals eine praktikable Methode etabliert werden, bei der bioptisch gewonnene Fibroblasten vom gleichen Patienten durch entsprechende Differenzierungsmedien in Fett-, Knochen- und Knorpelzellen umgewandelt werden konnten. Die erzielten Befunde belegen jedenfalls zweifelsfrei, dass unter den gewählten Differenzierungsbedingungen in vitro o.g. Zellpopulationen herzustellen sind und damit u.a. ein schneller, kostengünstiger und unkomplizierter Weg für das Züchten von potenten autologen (vom gleichen Patienten) Zellen für einen Gewebeersatz gefunden wurde.

Somit belegen die zu der vorliegenden Erfindung führenden Befunde, dass kultivierte Fibroblasten die Potenz besitzen, als sehr unkompliziert beschaffbare Quelle, z.B. für ein "Tissue Engineering", genutzt zu werden. Alle bisher bekannten Ansätze gehen von der Verwendung von adulten oder auch embryonalen Stammzellen aus, deren Gewinnung nicht nur ethische Hindernisse, wie bei den embryonalen Stammzellen, besitzt sondern auch im Falle der adulten Stammzellen aufwendig, teuer und für den Patienten nicht ohne Risiko ist.

An Hand der aufgeführten Beispiele konnten die Erfinder klar zeigen, dass entweder primäre kultivierte Fibroblasten in der Lage sind, eine Transdifferenzierung mit dem Ergebnis der Ausbildung eines anderen mesenchymalen Phänotyps zu durchlaufen, oder dass sich möglicherweise in den primären Fibroblastenkulturen, und dies auch nach mehrfacher Passagierung der Zellkulturen, dermale Stammzellen/Vorläuferzellen befinden, die sich dann ihrerseits bei entsprechenden Differenzierungsbedingungen in die jeweiligen Phänotypen differenzieren lassen.

Der in der vorliegenden Anmeldung verwendete Ausdruck "tierische Zellen", umfasst alle tierischen Zellen, vorzugsweise Säugerzellen, z.B. von Menschen, Ratte, Hamster, Schwein, Rind oder Kaninchen, wobei menschliche Zellen am meisten bevorzugt sind.

Vorzugsweise handelt es sich bei den Fibroblasten (bzw. den Fibroblasten der Fibroblastenkultur) um Fibroblasten, die aus Biopsien von entsprechendem Gewebe, z.B. Bindegewebe der Haut, der Vorhaut, der Kopfhaut oder auch Bindegewebe anderen Ursprungs, das als Hauptzellbestandteil Fibroblasten aufweist, gewonnen wurden. Bei der Biopsie handelt es sich z.B. um eine Nadel-, Saug-, Stanz-, Exzisions-oder Feinnadel-Biopsie, wobei die Stanz- und Exzisionsbiopsie bevorzugt ist.

In einer bevorzugten Verwendung handelt es sich bei den Fibroblasten um dermale Fibroblasten.

In einer weiteren bevorzugten Verwendung handelt es sich bei den differenzierten tierischen Zellen um Fettzellen, Knorpelzellen oder Knochenzellen.

In einer bevorzugten Ausführungsform der Erfindung erfolgt die Gewinnung der differenzierten tierischen Zellen dadurch, dass die Fibroblasten in einem Medium kultiviert werden, das die für die Differenzierung zu den jeweiligen Zellen notwendigen Wachstumsfaktoren enthält. Der Fachmann kennt geeignete Züchtungsverfahren und Medien, um Fibroblasten kultivieren zu können (Freshney RI Culture of animal cells. A manual of technique. 2nd Edition ed, Wiley-Liss. New York (19879). Das "Grundmedium", das u.a. Wachstumsfaktoren enthält, kann jedes Medium sein, das üblicherweise für die Züchtung von Tierzellen verwendet wird. Die gewünschten Zellen werden in dem vorstehenden Medium unter geeigneten Bedingungen, gegebenenfalls unter (teilweiser) Erneuerung des Mediums in geeigneten Zeitabständen, gezüchtet. Geeignete Bedingungen, beispielsweise hinsichtlich geeigneten Behältern, Temperatur, relativer Luftfeuchte, O₂-Gehalt und CO₂-Gehalt der Gasphase sind dem Fachmann bekannt. Vorzugsweise werden die Zellen in dem vorstehenden Medium unter den folgenden Bedingungen kultiviert: (a) 36-38°C, vorzugsweise 37°C, (b) 90-100% rel. Luftfeuchte, vorzugsweise 95% rel. Luftfeuchte, (c) 5-15% O₂, vorzugsweise 10% O₂ und (d) 3 bis 10% CO₂, vorzugsweise 5% bis 7% CO₂. Der Fachmann kann auch die gewünschte Steuerung der Differenzierung der tierischen Zellen anhand üblicher Kriterien (morphologische Kriterien, Anwesenheit bzw. Abwesenheit spezifischer Oberflächenproteine etc.) überwachen.

Durch Zugabe entsprechender Verbindungen zu dem Kulturmedium, z.B. Zytokine, kann der Erhalt des gewünschten Zelltyps gesteuert werden.

Es hat sich für die adipogene Differenzierung von tierischen Fibroblasten als bevorzugt herausgestellt, diese zuerst in einem "Induktionsmedium" genannten Medium für 2-5 Tage zu kultivieren, bevor sie in einem "Unterhaltsmedium" genannten Medium 2-5 Tage weiterkultiviert werden. Dann wurde wieder zum Induktionsmedium gewechselt. Ein 2 bis 5-maliger Wechsel zwischen Induktions- und Unterhaltsmedium hat sich bewährt. Nach dem letzten Wechsel sollte noch ca. 5 bis 10 Tage in Unterhaltsmedium weiterkultiviert werden.

Das "Induktionsmedium" hat bevorzugt folgende Zusammensetzung:
- high glucose DMEM Medium, ergänzt mit
- 0,1 - 3 *µ*M Dexamethason (bevorzugt 0,5-2 *µ*M, ganz bevorzugt 1 *µ*M)
- 0,1 - 0,4 mM Indomethazin (bevorzugt 0,2 *µ*M)
- 0,005 - 0,05 mg/ml TGFβ (bevorzugt 0,01 mg/ml)
- 0,005 - 0,05 mg/ml Insulin (bevorzugt 0,01 mg/ml)
- 0,1 - 2 mM 3-Isobutil-I-Methylxantin (0,5 mM)
- 1 - 5*µ*g/ ml Transferrin (bevorzugt 2 *µ*g/ml)
- 5 - 20% fötales Kälberserum (bevorzugt 10%)
- 0,005 - 0,1 U/ml Pencillin (bevorzugt 0,05 U/ml)
- 0,005 - 0,1 *µ*g/ml Streptomycin (bevorzugt 0,05 *µ*g/ml)

Das "Unterhaltsmedium" hat bevorzugt folgende Zusammensetzung:
- high glucose DMEM Medium, ergänzt mit
- 0,005 - 0,05 µg/ml TGFβ3 (bevorzugt 0,01 *µ*g/ml)
- 0,005 - 0,05 mg/ml Insulin (bevorzugt 0,01 *µ*g/ml)
- 5 - 20% fötales Kälberserum (bevorzugt 10 %)
- 0,005 - 0,1 U/ml Penicillin (bevorzugt 0,05 U/ml)
- 0,005 - 0,1 *µ*g/ml Streptomycin (bevorzugt 0,05 *µ*g/ml)

Für die osteogene Differenzierung von tierischen Fibroblasten hat sich folgende Vorgehensweise als bevorzugt herausgestellt.

Die Zellen werden in Zellkulturschalen in osteogenem Unterhaltsmedium [High Glukose-DMEM Medium, welches 3-8 *µ*g/ml Rinder-Insulin (bevorzugt 6-7*µ*g/ml), 4-9 *µ*g/ml Transferrin (bevorzugt 6-8 *µ*g/ml, ganz bevorzugt 7 *µ*g/ml), 4-9*µ*g/ml selenige Säure (bevorzugt 6-8 *µ*g/ml, ganz bevorzugt 7 *µ*g/ml), 4-9 *µ*g/ml Linolsäure (bevorzugt 6-8 *µ*g/ml, ganz bevorzugt 7 *µ*g/ml) und 0,5-3 % Rinderserum-Albumin (bevorzugt 1-2 %) enthielt], ausgesät und kultiviert. Nach 12-48 Stunden (bevorzugt 24 Stunden) Kultur wird das Medium durch osteogenes Induktionsmedium [High Glukose-DMEM Medium, welches 3-8 *µ*g/ml Rinder-Insulin (bevorzugt 6-7 *µ*g/ml), 4-9 *µ*g/ml Transferrin (bevorzugt 6-8 *µ*g/ml, ganz bevorzugt 7 *µ*g/ml), 4-9 *µ*g/ml selenige Säure (bevorzugt 6-8 *µ*g/ml, ganz bevorzugt 7 *µ*g/ml), 4-9 *µ*g/ml Linolsäure (bevorzugt 6-8 *µ*g/ml, ganz bevorzugt 7 *µ*g/ml), 0,5-3 % Rinderserum-Albumin (bevorzugt 1-2 %), 0,01-0,2 *µ*M Dexamethason (bevorzugt 0,1-0,15 *µ*M, ganz bevorzugt 0,12 *µ*M), 0,01-0,1 mM Ascorbinsäure-2-phosphat (bevorzugt 0,05 mM), 0,5-2 *µ*g/ml humanes Fibronektin (bevorzugt 1 *µ*g/ml) und 1-30 mM Glycerophosphat (bevorzugt 5-20 mM, ganz bevorzugt 10 mM) enthielt], für mindestens 2-4, bevorzugt 3 Wochen, kultiviert. Das osteogene Induktionsmedium wurde vorzugsweise alle zwei bis fünf, bevorzugt alle drei, Tage gewechselt.

Für die chondrogene Differenzierung von tierischen Fibroblasten hat sich folgende Vorgehensweise als bevorzugt herausgestellt.

Die Zellen werden als Pellets kultiviert, das normale Zellulturmedium entfernt und durch chondrogenes Induktionsmedium für 12-48, bevorzugt 24, Stunden ersetzt.

Das chondrogene Induktionsmedium weist bevorzugt folgende Zusammensetzung auf:
High Glukose-DMEM Medium, ergänzt mit
   0,1-0,2,*µ*M Dexamethason (bevorzugt 0,15,*µ*M)
   0,5-3 mM Natriumpyruvat (bevorzugt 1 mM),
   0,1-0,2 mM Ascorbinsäure-2-phosphat (bevorzugt 0,17 mM),
   0,1-1 mM Prolin (bevorzugt 0,4 mM),
   4-9 *µ*g/ml Rinder Insulin (bevorzugt 6-7 *µ*g/ml),
   4-9 *µ*g/ml Transferrin (bevorzugt 6-8 *µ*g/ml, ganz bevorzugt 7 *µ*g/ml)
   4-9 *µ*g/ml selenige Säure (bevorzugt 6-8 *µ*g/ml, ganz bevorzugt 7 *µ*g/ml),
   4-9 µg/ml Linolsäure (bevorzugt 6-8 *µ*g/ml, ganz bevorzugt 7 *µ*g/ml),
   1-2 % Rinderserum-Albumin (bevorzugt 1,25 %),
   0,01-0,05 *µ*g/ml TGF-β₃ (bevorzugt 0,02 *µ*g/ml)
   10-500 U/ml Penicillin (bevorzugt 50-200 U/ml, ganz bevorzugt 100 U/ml) und
   10-500 U/ml Streptomycin (bevorzugt 50-200 U/ml, ganz bevorzugt 100 U/ml).

Die Zellpellets wurden durch leichtes kurzes Schütteln abgelöst und für mindestens 1-5, bevorzugt 3, Wochen in chondrogenem Induktionsmedium kultiviert. Das chondrogene Induktionsmedium wurde vorzugsweise alle zwei bis fünf, bevorzugt alle drei, Tage gewechselt.

Die über die erfindungsgemäße Vorgehensweise erhältlichen differenzierten Zellen sind vor allem für die therapeutische Nutzung in der Wundheilung und der Transplantationsmedizin von Interesse.

Die Erfindung soll anhand der folgenden Beispiele näher erläutert werden.

### Methodenbeschreibung

### 1. Gewinnung humaner primärer Fibroblastenkulturen

Für die Untersuchungen fand menschliche Haut, die nach kosmetischen Operationen anfiel, Verwendung. In jedem Fall war das Einverständnis des Patienten eingeholt worden.

Hautbioptate wurden nach Entfernen des Fettgewebes in etwa 1 mm² große Stücke geschnitten, die dann auf 250 ml Polystyrol-Gewebekulturflaschen verteilt, für eine 1 h im Brutschrank angetrocknet und schließlich in Dulbecco's modifiziertem Eagle's Medium (DMEM), kultiviert, das mit 10 % fetalem Kälberserum (FCS), L-Glutamin (292 *µ*g/ml), Ascorbinsäure (50 *µ*g/ml), Penicillin (200 IE/ml) und Streptomycin (200 mg/ml) ergänzt ist. Die Kultivierung erfolgte bei 37 °C und 5 % (v/v) CO₂-Gehalt und 95 % (v/v) relativer Luftfeuchtigkeit. Nach 2-3 Wochen wuchsen aus den Gewebestücken zunächst Keratinozyten und später Fibroblasten aus. Nach Erreichen eines konfluenten Zellrasens wurden die Zellen mit PBS gespült, 2 min mit 0,05 % Trypsin/ 0,02 % (w/v) EDTA inkubiert und danach abgelöst. Die Wirkung des Trypsins wurde durch Zugabe von Medium abgestoppt, die Zellen auf 2-4 neue Flaschen verteilt und weiterkultiviert. Nach 1-2 Wochen erreichten die Fibroblasten erneut Konfluenz und wurden dann, wie oben beschrieben, passagiert und subkultiviert.

Nach der zweiten Passage waren mittels morphologischer und immunhistochemischer Überprüfung nur noch Fibroblasten in der Kultur nachweisbar.

### 2. Isolierung humaner mesenchymaler Stammzellen aus dem Knochenmark

Humane mesenchymale Stammzellen (MSC) wurden entweder mittels Feinnadelaspiration des Knochenmarks des Beckenkammes von freiwilligen gesunden Spendern gewonnen, oder von der Firma Cambrex (Verviers, Belgien) bezogen.

Das Feinnadelaspirat wurde in 1 bis 2 ml Iscove's MDM Medium, welches 2% fetales Rinderserum enthielt, aufgenommen. Die Zellsuspension wurde in Zellkulturflaschen überführt und für 7 Tage in Poietics^{tm} Mesenchymal Stem Cell Growth Medium (MSCGM^{tm}, Cambrex) im Brutschrank bei 37°C und 5 % (v/v) CO₂-Gehalt und 95 % (v/v) relativer Luftfeuchtigkeit kultiviert. Alle nichtadhärenten Zellen wurden verworfen und die adhärenten Zellen in einem weiteren Schritt bis zur Konfluenz des Zellrasens unter gleichen Bedingungen kultiviert.

Nach Trypsinierung zeigten alle Zellen in der Flowzytometrie eine positive Reaktion auf die bekannten und akzeptierten mesenchymalen Stammzellmarker CD-44, CD-90 und CD-105.

### 3. Nachweis der Fettablagerung (adipogene Differenzierung)

Der Nachweis der Fetttröpfchenbildung in den adipogen differenzierten Zellen erfolgte entsprechend literaturbekannter Angaben mittels Oil Red O Anfärbung.

Eine Oil Red O Stocklösung wurde durch Lösung von 0,5 g Oil Red O in 100 ml 99%igem Isopropanol hergestellt. Für die Arbeitslösung wurden 6 Volumenteile der Stocklösung mit 4 Teilen destillierten Wassers verdünnt und danach gefiltert. Die auf Objektträgern kultivierten Zellen wurden für 7 min mittels 10% Paraformaldehyd in PBS bei Raumtemperatur fixiert und in destilliertem Wasser gespült. Danach wurden die Objektträger in einer wässrigen 60%igen Isopropanollösung für 5 min bei Raumtemperatur inkubiert und mit destilliertem Wasser gespült. Die Objektträger wurden danach für 10 min in die Oil Red O Arbeitslösung überführt, kurz mit 60%igem Isopropanol inkubiert und mit destilliertem Wasser mehrfach gewaschen. Die Zellkerne wurden mit Haematoxilin gegengefärbt.

### 4. Nachweis des abgelagerten Calziumphosphates (osteogene Differenzierung)

Der Nachweis der Calziumphosphatablagerungen in den Zellen nach osteogener Differenzierung wurde entsprechend der von Kossa beschriebenen Methodik durchgeführt (von Kossa Über die im Organismus künstlicherzeugbaren Verkalkungen. Beitr Pathol Anat Alig Pathol (1901); 29: 162-202).

Die auf Objektträgern kultivierten Zellen wurden in 1% Paraformaldehyd in PBS für 7 min fixiert. Nach kurzem Waschen der Objektträger in destilliertem Wasser, wurden die Objektträger für 30 min in einer 5%igen Silbernitratlösung inkubiert und im Anschluss einer intensiven Lichtexposition ausgesetzt. Danach wurden die Objektträger in destilliertem Wasser gewaschen und 3 min in einer 1%igen wässrigen Gallussäure inkubiert. Nach mehrfachem Waschen der Objektträger in destilliertem Wasser, wurden diese für 5 min in 5%iger wässriger Thiosulfatlösung inkubiert, mehrfach mit destilliertem Wasser gewaschen und für die mikroskopische Auswertung getrocknet.

### 5. Nachweis der alkalischen Phosphatase Aktivität

Der Nachweis der Aktivität der alkalischen Phosphatase erfolgte mit dem Alkalische Phosphatase-Kit (Sigma; 86-R) nach den Angaben des Herstellers. Wlodarski und Reddi konnten zweifelsfrei belegen, dass die Aktivität der Alkalischen Phophatase als ein Marker der osteogenen Differenzierung aufzufassen ist (Wlodarski KH and Reddi AH Alkaline phosphatase as a marker of osteoinductive cells. Cacif Tissue (1986) 39: 382-385).

### 6. Immunfluoreszenz

Sektionsschnitte von Zellpeletts nach chondrogener Differenzierung wurden mittels Kollagen Typ II Antikörpern auf die für Chondrozyten typische Expression von Kollagen Typ II untersucht.

Die Schnitte wurden in 2%igem Paraformaldehyd für 10 min bei Raumtemperatur fixiert.Die Schnitte wurden danach mit 2 mg/ml Pepsin in 0,01 N HCl für 45 min bei 37°C angedaut, um den Antikörper eine Erkennung und Bindung des intrazellulären Kollagen Typ II zu ermöglichen. Die Schnitte wurden mit 5% Ziegenserum und 1 % Rinderserumalbumin in PBS (Blocking Puffer) für 30 min bei Raumtemperatur blockiert. Der Maus anti-human Kollagen II Antikörper (MAB8887, Chemicon, Hofheim, Germany) wurde auf 4 *µ*g/ml in Blocking Puffer verdünnt und damit die Schnitte über Nacht bei 4°C inkubiert. Die Isotypen-Kontrolle mit MOPC21 (Sigma, Taufkirchen, Germany) wurde unter gleichen Bedingungen behandelt. Die Schnitte wurden am nächsten Tag mehrfach mit PBS gewaschen. Der Nachweis der gebundenen Kollagen Typ II Primärantikörper erfolgte nach 1 Stunde Inkubation bei Raumtemperatur mit dem 1:1000 in Blocking Puffer plus 1 *µ*g/ml 4',6-diamidino-2-phenylindole (DAPI, Sigma) verdünntem Sekundärantikörper Alexa 488-labeled goatanti-mouse Antikörper (Molecular Probes, Leiden, The Netherlands). Nach mehreren PBS Waschschritten wurden die gefärbten Schnitte an einem Nikon Eclipse 800 Mikroskop unter Zuhilfenehme einer Digitalkammera (Nikon, Düsseldorf, Germany) und einer Bilanalysesoftware (Lucia G, Laboratory imaging) ausgewertet.

### 7. RNA Isolation und Reverse Transkription

Die Total-RNA der Zellkulturen wurde entsprechend den Angaben der Hersteller mittels Qiagen RNAeasy Säulen isoliert. Für die Reverse Transkription wurden jeweils 1 *µ*g Total-RNA benutzt und diese mit Hilfe des Invitrogen Superscript II Kits entsprechend des Protokolls des Herstellers durchgeführt. Die Effizienz der Reversen Transkription wurde entsprechend früherer Erfahrungen als 100% angenommen.

### 8. Quantitative PCR

Die quantitativen PCR Experimente wurden jeweils in Dreifachbestimmung für jede Probe, mit Hilfe des Quiagen Quantitec SYBRgreen Kitts, nach den Angaben des Herstellers, durchgeführt. Für jede PCR Reaktion wurden 10 ng cDNA verwendet. Die Primer wurden mit Hilfe der Primer Express Software von Applied Biosystems designed. Folgende Primer wurden benutzt:
1. Adipsin forward primer 5' CCGACACCATCGACCACG 3'
2. Adipsin reverse primer 5' AGAGTTCCCGGTGCCACG 3'
Die Ergebnisse wurden mittels MRPS26 ribosomal protein RNA normalisiert.
1. MRPS26 forward primer 5' CCGACACCATCGACCACG 3'
2. MRPS26 reverse primer 5' TTCACATACAGCTTGGGAAGCA 5'

Nachfolgend sind die genutzten Zyklusparameter aufgeführt:
50°C für 2 min, 95°C für 15 min gefolgt von 40 Zyklen bei 94°C für 15 sec; 55°C für 30 sec; 72°C für 30 sec.

Die quantitativen PCR Experimente wurden an einem ABI Biosystems Genmap 5700 Sequence Detection System durchgeführt. Die Ergebnisse wurden mit der mitgelieferten SDS Software analysiert. Die Spezifität der Amplikationsreaktionen wurden mittels der melting curve Analyse entsprechend dem Protokoll von ABI Biosystems beurteilt.

### Beispiel 1

### Differenzierung von Fibroblasten und MSC in Adipocyten

Humane MSC wurden generell als Positivkontrolle für die Differenzierung von Fibroblasten mitgeführt.

Die Differenzierung in Adipocyten ist durch eine Akkumulation von Fetttröpfchen in den Zellen und durch die Hochregulation von spezifischen Fettzellmarkern, wie z.B. dem Adipsin, charakterisiert. Eine Adipocytendifferenzierung von MSC kann einmal dadurch induziert werden, wenn die Zellen längere Zeit in einem überkonfluenten Stadium kultiviert werden, oder durch eine Stimulation mit Insulin (Prockop DJ Marrow stromal cells as stem cells for nonhemapoietic tissues. Science (1997) 276: 71-74; Pittenger MF et al. Human mesenchymal stem cells: progenitor cells for cartilage, bone, fat and stroma. Curr Top Microbiol Immunol (2000) 251: 3-11).

Die adipogene Differenzierung der mesenchymalen Stammzellen des Knochenmarks und der humanen primären Fibroblasten wurde wie folgt durchgeführt:

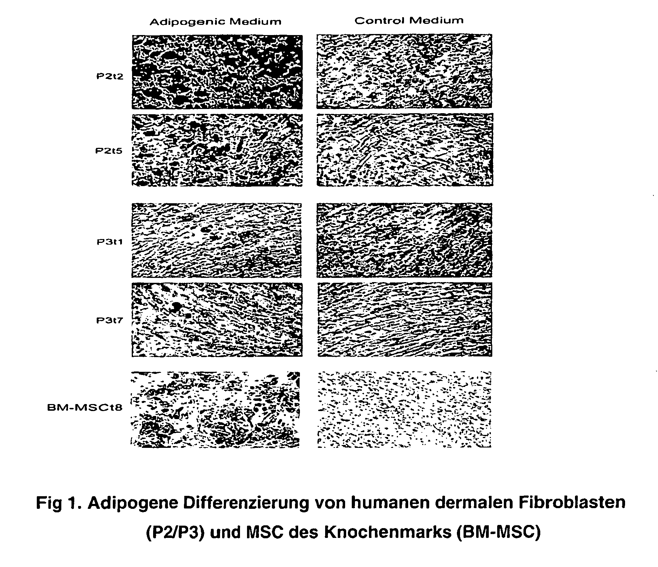

Fibroblasten bzw. MSC wurden für eine adipogene Differenzierung in Nunc Lab-Tek-I oder II Gewebekulturslides oder in 6-well Zellkulturplatten in einer Dichte von 2,1x10⁴ Zellen pro cm² ausgesäht. Die Zellen wurden bis zur Konfluenz in MSCGM^{tm} Medium im Brutschrank bei 37°C und 5 % (v/v) CO₂-Gehalt und 95 % (v/v) relativer Luftfeuchtigkeit kultiviert. Bei 100iger Konfluenz des Zellrasens wurde die adipogene Differenzierung mit einem so genannten Induktionsmedium eingeleitet. Mit dem adipogenen Induktionsmedium, welches High-glucose DMEM Medium zuzüglich 1 *µ*M Dexamethason, 0,2 mM Indomethazin, 0,01. mg/ml TGFβ, 0,01 mg/ml Insulin, 0,5 mM 3-Isobutil-I-Methylxanthin, 2 *µ*g/ml Transferrin, 10% fetales Kälberserum (getestet auf Wachstums-induzierende Aktivität), 0,05 U/ml Penicillin und 0,05 *µ*g/ml Streptomycin enthielt, wurden die Zellen für 3 Tage kultiviert. Danach wurde das Medium durch das adipogene Unterhaltmedium (High-glucose DMEM, welches 0,01 *µ*g/ml TGFβ3 (R&D Systems, Wiesbaden-Nordenstadt, Germany), 0,01 mg/ml Insulin, 10% fetales Kälberserum, 0,05 U/ml Penicillin und 0,05 *µ*g/ml Streptomycin enthielt), ersetzt. Dieser Wechsel von Induktionsmedium in Unterhaltmedium und umgekehrt wurde dreimal wiederholt. Nach dem letzten Wechsel wurden die Zellen für 7 Tage im Unterhaltmedium kultiviert. Nach diesem Zeitpunkt wurden die Zellen (Fibroblasten, MSC) analysiert. Kontrollkulturen wurden identisch ausgesät und die gesamte Zeit in adipogenen Unterhaltmedium kultiviert. (Fig. 1)

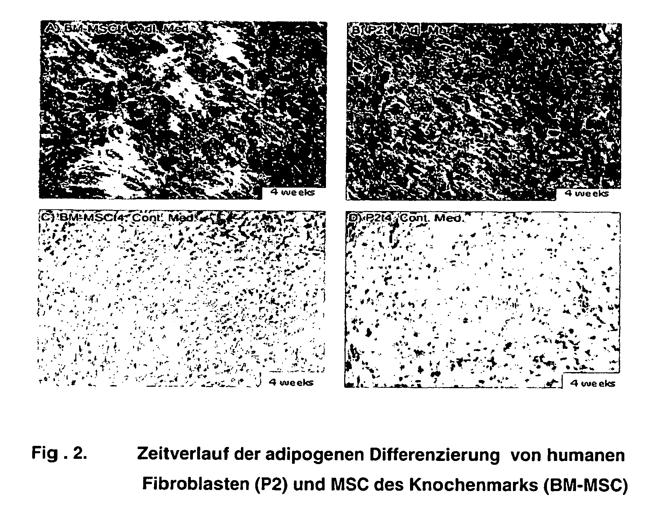

Figur 1 präsentiert auf der linken Seite Zellen 3 Wochen nach adipogener Differenzierung mit adipogenem Medium (= Kombination Induktionsmedium/Unterhaltsmedium) und auf der rechten Seite Zellen, die mit Kontrollmedium (nur adipogenes Unterhaltsmedium; Negativkontrolle) gleichfalls 3 Wochen kultiviert wurden. Der Lipidtröpfchennachweis mittels Oil-Red O zeigte eine stark positive Reaktion bei den differenzierten Fibroblasten des Patienten P2 und in deutlich abgeschwächter Form des Patienten P3, auch in Abhängigkeit von der jeweiligen Passagezahl(t1, t2, t5, t7). Die adipogene Differenzierung der mesenchymalen Stammzellen des Knochenmarks (BM-MSC) zeigte gleichfalls eine deutliche Ablagerung von rotgefärbten Fetttröpfchen. Die in der rechten Reihe dargestellten Zellen (ohne Kultivierung im adipogenen Induktionsmedium; Negativkontrolle) zeigten keinen positiven Fetttröpfchennachweis. Die Zellkerne wurden mittels HE blau gefärbt. Wie aus der Abbildung 1 weiterhin ersichtlich ist, existieren individuelle Unterschiede einzelner Patienten-Fibroblastenkulturen in ihrer Differenzierungspotenz, da in der Zellkultur des Patienten P3 wesentlich weniger Adipozyten nachweisbar waren. Das Alter dieses Patienten war 32 Jahre im Vergleich zu Patient P2 mit 63 Jahren. Im folgenden sollte die Zeitabhängigkeit der adipogenen Differenzierung untersucht werden. Ein Fetttröpfchennachweis war unmittelbar nach Zugabe des adipogenen Mediums am Tage 0, am Tag 3 und am Tag 8 negativ. Am Tag 13 der Kultivierung mit adipogenem Medium war sowohl in der Fibroblastenkultur, als auch in der MSC Kultur eine positive Anfärbung sichtbar. Nach 4-wöchiger Kultivierung der Zellen mit adipogenem Medium war eine deutliche positive Anfärbung (intrazelluläre Lipidablagerung) zu sehen (Fig. 2)

Die obere Spalte der Fig. 2 repräsentiert rechts adipogen differenzierte Fibroblasten P2 der Passage 4 und links mesenchymale Stammzellen BM-MSC der Passage 4. In der unteren Reihe sind die entsprechenden Kontrollen (ohne adipogenes Induktionsmedium) dargestellt. Die Zellkerne der Kulturen wurden mit HE gegengefärbt.

Als weiteren Nachweis der stattgefundenen adipogenen Differenzierung wurde mittels quantitativer PCR die spezifische mRNA Expression des Adipozytenspezifischen Proteins Adipsin bestimmt.

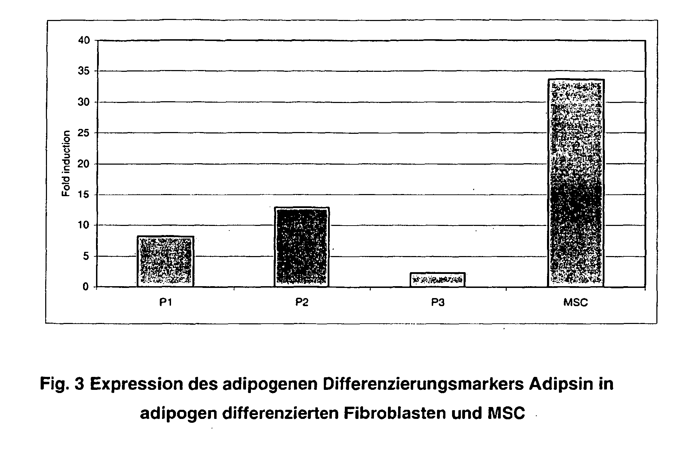

In Fig. 3 ist das Verhältnis der Adipsin mRNA Expression von unterschiedlichen Fibroblastenkulturen (P1, P2, P3) und von mesenchymalen Stammzellen (MSC) nach adipogener Differenzierung im Verhältnis zu den entsprechenden Kontrollkulturen (ohne adipogene Differenzierung) als mehrfache Induktion dargestellt. Wie aus der Abbildung zu ersehen ist, zeigen adipogen differenzierte MSC einen über 35-fachen Anstieg der Adipsin mRNA Expression. Auch bei allen untersuchten Fibroblasten nach adipogener Differenzierung war eine deutliche Expressionssteigerung bis über das 12-fache bei Patient P2 objektivierbar.

Zusammenfassend kann also festgestellt werden, dass sich ähnlich wie mesenchymale Stammzellen auch primäre Fibroblasten in Adipozyten differenzieren lassen. Offensichtlich scheint eine Abhängigkeit im Ausmaß der Differenzierung vom Alter des Patienten und von der Passagezahl zu existieren.

### Beispiel 2

### Differenzierung von Fibroblasten und MSC in Osteoblasten

Humane MSC wurden generell als Positivkontrolle für die Differenzierung von Fibroblasten mitgeführt.

Die osteogene Differenzierung der mesenchymalen Stammzellen des Knochenmarks und der humanen primären Fibroblasten wurden wie folgt durchgeführt:

Die Zellen wurden in Zellkulturschalen in einer Zelldichte von 3,1x10³ pro cm² für 24 Stunden in osteogenem Unterhaltsmedium (High Glukose-DMEM Medium, welches 6,5 *µ*g/ml Rinder-Insulin, 7 *µ*g/ml Transferrin, 7 *µ*g/ml selenige Säure, 7 *µ*g/ml Linolsäure und 1,25 Rinderserum Albumin enthielt), ausgesät. Die Kultivierung erfolgte in einem Brutschrank bei 37°C und 5% CO₂. Nach 24 Stunden Kultur wurde das Medium durch osteogenes Induktionsmedium (High Glukose-DMEM Medium, welches 6,5 *µ*g/ml Rinder-Insulin, 7 *µ*g/ml Transferrin, 7 *µ*g/ml selenige Säure, 7 *µ*g/ml Linolsäure, 1,25 Rinderserum-Albumin, 0,12 *µ*M Dexamethason, 0,05 mM Ascorbinsäure-2-phosphat, 1 *µ*g/ml humanes Fibronektin und 10 mM Glycerophosphat enthielt), für mindestens 3 Wochen kultiviert. Das osteogene Induktionsmedium wurde alle drei Tage gewechselt. Die Zellen der Negativkontrolle wurden nur in osteogenem Unterhaltsmedium über den gleichen Zeitraum und unter identischen Bedingungen kultiviert.

Die stattgefundene Differenzierung in Knochenzellen (Osteoblasten) ist dadurch charakterisiert, dass es zu einer Akkumulation von Calziumphosphat und zu einer erhöhten Expression des Enzyms Alkalische Phosphatase kommt. Mit Hilfe dieser international akzeptierten Marker sollte überprüft werden, ob ähnlich wie die adipogene Differenzierung auch eine osteogene Differenzierung von primären humanen Fibroblasten möglich ist.

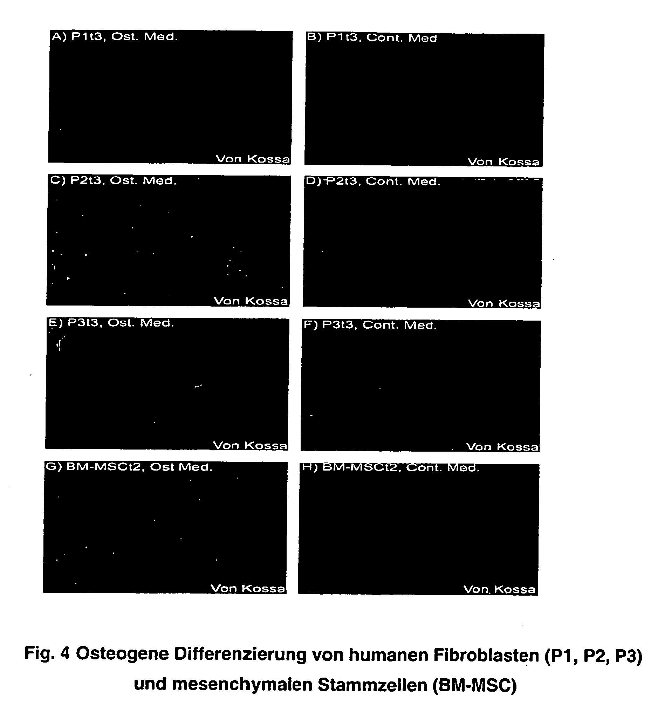

In Fig. 4 ist das Ergebnis der osteogenen Differenzierung von Fibroblasten und mesenchymalen Stammzellen an Hand des positiven Nachweises der Ablagerung von Calziumphosphat bei von Kossa-Färbung (braun/schwarze Färbung) dargestellt. Bei den Fibroblastenkulturen der Patienten P1 und P2 konnte wie bei den mesenchymalen Stammzellen eine massive Ablagerung von Calziumphosphat nachgewiesen werden. Dieser deutliche Nachweis gelang bei den Fibroblasten des Patienten P2 nicht im ausreichenden Umfang.

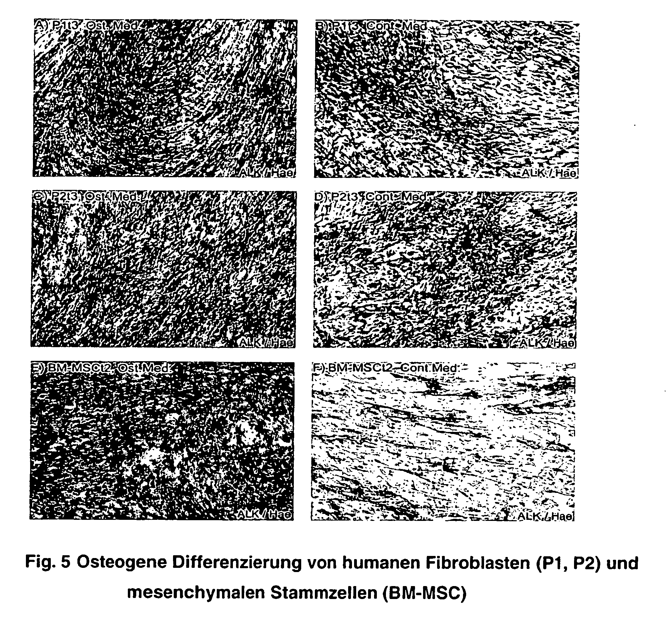

In der Fig. 5 ist die osteogene Differenzierung von Fibroblasten und mesenchymalen Stammzellen an Hand der Erhöhung der Expression/Aktivität der Alkalischen Phosphatase dargestellt. Jede Zelle besitzt einen gewissen Basislevel an alkalischer Phosphataseaktivität, aber erst nach osteogener Differenzierung nimmt die Expression und Aktivität dieses Enzyms stark zu.

Die in Fig. 5 dargestellte Färbung der alkalischen Phosphatase nach osteogener Differenzierung und den entsprechenden Kontrollen wurde nach einer Differenzierungszeit von 3 Wochen durchgeführt und ist als rot/violette Färbung sichtbar. Auf der linken Seite sind die osteogen differenzierten Fibroblasten (P1, P2) und mesenchymale Stammzellen (BM-MSC) als Positivkontrolle dargestellt. Es ist deutlich ersichtlich, dass die osteogen differenzierten Zellen (links) im Vergleich zu den entsprechenden Kontrollzellen (rechts) eine signifikant höhere alkalische Phosphataseaktivität aufweisen. Die Zellkerne wurden mit HE gegengefärbt.

Diese Daten belegen eindeutig die Tatsache, dass sich humane primäre Fibroblasten unter den angegebenen Differenzierungsbedingungen in Knochenzellen differenzieren lassen.

### Beispiel 3

### Differenzierung von Fibroblasten und MSC in Chondrozyten

Chondrogene Differenzierung von z.B. mesenchymalen Stammzellen ist durch die Hochregulation verschiedener Proteoglycane, wie z.B. des Aggregans und des für Knorpelzellen typische Kollagen Typ II charakterisiert. An Hand dieses Beispiels soll nachgewiesen werden, dass Kontrollfibroblasten das Kollagen Typ II nicht exprimieren, aber nach chondrogener Differenzierung dieses spezifische Protein nachweisbar ist.

Die chondrogene Differenzierung der mesenchymalen Stammzellen des Knochenmarks und der humanen primären Fibroblasten wurde wie folgt durchgeführt:

Die Zellen wurden als Pellets von ca. 2,5x10⁵ Zellen in Polypropylen Tubes (sterile Eppendorf caps) kultiviert. Um eine Pelletformation zu erreichen, wurden die Zellen für 3 min bei 150x g zentrifugiert, das normale Zellulturmedium entfernt und durch chondrogenes Induktionsmedium für 24 Stunden ersetzt. Das chondrogene Induktionsmedium bestand aus High Glukose-DMEM Medium, welches 0,15 *µ*M Dexamethason, 1 mM Natriumpyruvat, 0,17 mM Ascorbinsäure-2-phosphat, 0,4 mM Prolin, 6,5 *µ*g/ml Rinder Insulin, 7 *µ*g/ml Transferrin, 7 *µ*g/ml selenige Säure, 7 *µ*g/ml . Linolsäure, 1,25 Rinderserum Albumin, 0,02 *µ*g/ml TGF-β₃ (R&D Systems, Germany), 100 U/ml Penicillin und 100 U/ml Streptomycin. Die Zellpellets wurden durch leichtes kurzes Schütteln abgelöst und für mindestens 3 Wochen in chondrogenem Induktionsmedium bei 37°C, 5% CO₂ kultiviert. Das chondrogene Induktionsmedium wurde alle drei Tage gewechselt. Kontrollkulturen wurden mit DMEM Medium, welches 10% fetales Kälberserum enthielt, kultiviert.

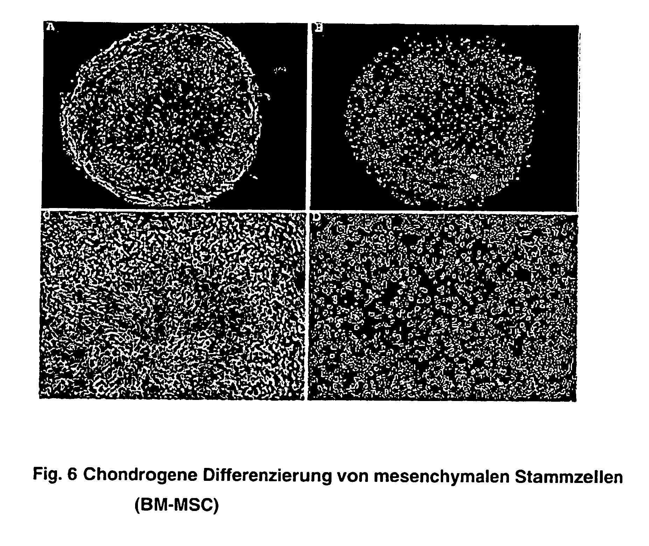

Mesenchymale Stammzellkulturen (BM-MSC) wurden unter chondrogenen Differenzierungsbedingungen (Fig. 6A und B) kultiviert, von den Zellpeletts Schnitte angefertigt und mit Kollagen Typ II Antikörpern gefärbt. In den Bilder B und C der Fig. 6 sind die entsprechenden Kontrollzellen ohne chondrogene Differenzierung dargestellt. Die grüne Färbung stellt das durch den spezifischen Kollagen Typ II Antikörper erkannte Kollagen dar und die blaue Färbung widerspiegelt die mit DAPI gegengefärbten Zellkerne. Die Vergrößerung der Bilder A und B beträgt 40x und der Bilder C und D 80x.

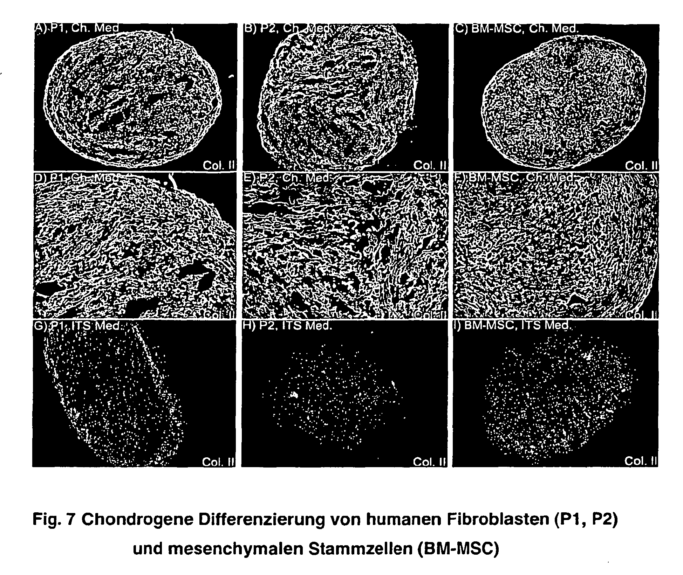

In Fig. 7 ist die chondrogene Differenzierung von Fibroblasten der Patienten P1 und P2, von mesenchymalen Stammzellen (BM-MSC) im Vergleich zu den entsprechenden Kontrollen dargestellt. Die Bilder A, B, C zeigen die immunhistochemische Anfärbung mit einem Kollagen Typ II spezifischen Antikörper bei einer Vergrößerung von 40x und die Bilder D, E und F bei einer Vergrößerung von 80x. Die Bilder G, H und I repräsentieren die entsprechenden Kontrollen (ohne chondrogenes Differenzierungsmedium) in der Vergrößerung 40x. Wie aus der Fig. 7 ersichtlich, sind sowohl mesenchymale Stammzellen als auch Fibroblasten in der Lage, unter chondrogenen Differenzierungsbedingungen das für Knorpelzellen typische und selektive Kollagen Typ II zu exprimieren (grüne Fluoreszenz). Der in der Kontrolle (Bild G, H und I) verwendete isotypenspezifische Antikörper zeigte keine unspezifische Bindung. Die Zellkerne der Schnitte wurden mit DAPI gegengefärbt und erscheinen blau.

## Patentansprüche

1. Verwendung von differenzierten Fibroblasten oder einer primären Fibroblastenkultur zur in-vitro Transdifferenzierung in Fett-, Knorpel- oder Knochenzellen.

2. Verwendung nach Anspruch 1, wobei die Fibroblasten oder die primäre Fibroblastenkultur aus Biopsiematerial gewonnen wurden.

3. Verwendung nach Anspruch 1 oder 2, wobei die Fibroblasten von Menschen, Ratte, Hamster, Schwein. Rind oder Kaninchen stammen.

4. Verwendung nach einem der Ansprüche 1 bis 3, wobei die Fibroblasten dermale Fibroblasten sind.

5. In-vitro Verfahren zur Gewinnung von Fettzellen aus differenzierten Fibroblasten oder einer primären Fibroblastenkultur, wobei die Fibroblasten zuerst in einem Kulturmedium, das mit Dexamethason, Indomethacin, TGFß, Insulin, 3-Isobutil-I-Methylxantin, Transferrin, fetalem Kälberserum, Penicillin und Streptomycin ergänzt ist, und dann in einem Kulturmedium, das mit TGFβ3, Insulin, fetalem Kälberserum, Penicillin und Streptomycin ergänzt ist, kultiviert werden, wobei zwischen den Kulturmedien ein 2- bis 5-maliger Wechsel stattfindet

6. In-vitro Verfahren zur Gewinnung von Knorpelzellen aus differenzierten Fibroblasten oder einer primären Fibroblastenkultur, wobei die Fibroblasten in einem Kulturmedium, das mit Dexamethason, Natriumpyruvat, Ascorbinsäure-2-phosphat, Prolin, Insulin, Transferrin, seleniger Säure, Linolsäure, Rinderserumalbumin, TGF-β3, Penicillin und Streptomycin ergänzt ist, kultiviert werden.

7. In-vitro Verfahren zur Gewinnung von Knochenzellen aus differenzierten Fibroblasten oder einer primären Fibroblastenkultur, wobei die Fibroblasten in einem Kulturmedium, das mit Insulin, Transferrin, seleniger Säure, Linolsäure und Rinderserum-Albumin ergänzt ist, ausgesät und mehrere Stunden kultiviert werden und danach in einem Kulturmedium, das mit Insulin, Transferrin, seleniger Säure, Linolsäure, Rinderserumalbumin, Dexamethason, Ascorbinsäure-2-phosphat, humanem Fibronektin und Glycerophoshat ergänzt ist, mehrere Wochen kultiviert werden.

8. Verfahren nach einem der Ansprüche 5 bis 7, wobei die Fibroblasten oder die primäre Fibroblastenkultur aus Biopsiematerial gewonnen wurden.

9. Verfahren nach einem der Ansprüche 5 bis 8, wobei die Fibroblasten von Menschen, Ratte, Hamster, Schwein, Rind oder Kaninchen stammen.

10. Verfahren nach einem der Ansprüche 5 bis 9, wobei die Fibroblasten dermale Fibroblasten sind.

## Claims

1. Use of differentiated fibroblasts or a primary fibroblast culture for the in vitro transdifferentiation in adipocytes, chondrocytes or bone cells.

2. Use according to claim 1, wherein the fibroblasts or the primary fibroblast culture were obtained from biopsy material.

3. Use according to claim 1 or 2, wherein the fibroblasts originate from man, rat, hamster, pig, cattle or rabbit.

4. Use according to any of claims 1 to 3, wherein the fibroblasts are dermal fibroblasts.

5. An in vitro process for obtaining adipocytes from differentiated fibroblasts or a primary fibroblast culture, wherein the fibroblasts are at first cultivated in a culture medium that is supplemented by dexamethasone, indomethacin, TGFβ, insulin, 3-isobutyl-methyl xantine, transferrin, fetal calf serum, penicillin and streptomycin and then in a culture medium that is supplemented by TGFβ3, insulin, fetal calf serum, penicillin and streptomycin, 2 to 5 changes taking place between the culture media.

6. The in vitro process for obtaining chondrocytes from differentiated fibroblasts or a primary fibroblast culture, the fibroblasts being cultivated in a culture medium that is supplemented by dexamethasone, sodium pyruvate, ascorbic acid-2-phosphate, proline, insulin, transferrin, selenous acid, linoleic acid, bovine serum albumin, TGF-β3, penicillin and streptomycin.

7. The in vitro process for obtaining bone cells from differentiated fibroblasts or a primary fibroblast culture, wherein the fibroblasts are sown in a culture medium that is supplemented by insulin, transferrin, selenous acid, linoleic acid and bovine serum albumin and are cultivated for several hours and are then cultivated in a culture medium that is supplemented by insulin, transferrin, selenous acid, linoleic acid, bovine serum albumin, dexamethasone, ascorbic acid-2-phosphate, human fibronectin and glycerophosphate, for several weeks.

8. The process according to any of claims 5 to 7, wherein fibroblasts or the primary fibroblast culture are obtained from biopsy material.

9. The process according to any of claims 5 to 8, wherein the fibroblasts originate from man, rat, hamster, pig, cattle or rabbit.

10. The process according to any of claims 5 to 9, wherein the fibroblasts are dermal fibroblasts.

## Revendications

1. Utilisation de fibroblastes différenciés ou bien une culture primaire de fibroblastes pour la trans-différenciation in vitro en adipocytes, chondrocytes ou cellules d'os,

2. Utilisation selon la revendication 1, **caractérisée en ce que** les fibroblastes ou bien la culture primaire de fibroblastes sont obtenus à partir d'un matériau de biopsie.

3. Utilisation selon la revendication 1 ou 2, **caractérisée en ce que** les fibroblastes proviennent des humains, des rats, des hamsters, des porcs, des bovins ou des chiens.

4. Utilisation selon l'une des revendications 1 à 3, **caractérisée en ce que** les fibroblastes sont des fibroblastes du derme.

5. Procédé in vitro pour obtenir des adipocytes à partir de fibroblastes différenciés ou bien d'une culture primaire de fibroblastes, dans lequel les fibroblastes sont cultivés d'abord dans un milieu de culture qui est complété avec le dexaéthasone, l'indométacine, TGFB, l'insuline, la 3-isobutyl-1-méthylxantine, la transferrine, le sérum foetal de veau, la pénicilline et la streptomycine, puis dans un milieu de culture qui est complété par TGFB3, l'insuline, le sérum foetal de veau, la pénicilline et la streptomycine, où se produit entre les milieux de culture une transformation 2- à 5-fois.

6. Procédé in vitro pour obtenir des chondrocytes à partir de fibroblastes différenciés ou bien une culture primaire de fibroblastes, **caractérisé en ce que** les fibroblastes sont cultivés dans un milieu de culture qui est complété avec la dexaméthasone, le pyruvate de sodium, l'acide ascorbique-2-phosphate, la proline, l'insuline, la transferrine, l'acide sélénique, l'acide linoléique, l'albumine de sérum de bovin, TGF-B3, la pénicilline et la streptomycine.

7. Procédé in vitro pour obtenir des cellules d'os à partir de fibroblastes différenciés ou d'une culture primaire de fibroblastes, **caractérisé en ce que** les fibroblastes sont semés et cultivés plusieurs heures dans un milieu de culture complété par l'insuline, la transferrine, l'acide sélénique, l'acide linoléique et l'albumine du sérum de bovin, puis ensuite cultivés durant plusieurs semaines dans un milieu de culture complété avec l'insuline, la transférine, l'acide sélénique, l'acide linoléique, l'albumine de sérum de bovin, la dexaméthasone, l'acide ascorbique-2-phosphate, la fibronectine humaine et le glycérophosphate.

8. Procédé selon l'une des revendications 5 à 7, **caractérisé en ce que** les fibroblastes ou la culture primaire de fibroblastes sont obtenus à partir d'un matériau de biopsie.

9. Procédé selon l'une des revendications 5 à 8, **caractérisé en ce que** les fibroblastes proviennent des humains, des rats, des hamsters, des porcs, des bovins ou des chiens.

10. Procédé selon l'une des revendications 5 à 9, **caractérisé en ce que** les fibroblastes sont les fibroblastes dermiques.
